# EUROPEAN PATENT SPECIFICATION

(11) **EP 0 508 311 B1**
(45) Date of publication and mention of the grant of the patent: **06.12.1995**
(21) Application number: 92105713.9
(22) Date of filing: 02.04.1992
(51) Int. Cl.: A61K 31/40, A61K 47/10, A61K 47/26, A61K 47/32

(54) **Injectable compositions containing 2-oxindole-1-carboxamide derivatives**
Injizierbare 2-Oxindol-1-carboxamidderivate enthaltende Arzneizubereitungen
Compositions injectable de dérivés du 2-oxindole-1-carboxamide

(30) Priority: 08.04.1991 DE 4111306
(43) Date of publication of application: 14.10.1992
(73) Proprietor: Heinrich Mack Nachf., D-89252 Illertissen (DE)
(72) Inventor: Fries, Walter, W-7918 Illertissen (DE); Pfitzner, Carl Jörg, W-7918 Illertissen (DE); Pfaff, Günther, Dr., W-7918 Illertissen (DE)
(74) Representative: Lederer, Franz, Dr.

(56) References cited:
- EP-A- 0 156 603
- EP-A- 0 337 628

## Description

Pharmaceutical compositions are often associated with the problem that it is intended to administer the therapeutically active agents parenterally, in which case it is necessary to produce a solution of the agent which is in the form of a solid. It is often wished in this connection to use water as solvent.

US Patent 4 556 672 describes 2-oxindole-1-carboxamide derivatives which are used in mammals and, in particular, in humans as analgesics. The anti-inflammatory action of these compounds, in particular, makes the 2-oxindole-1-carboxamide derivatives valuable therapeutically active agents.

It is necessary in the treatment to administer the agent in relatively high concentrations. However, it has been found that the solubility of these agents in water is only about 2 mg per ml at a pH of 7.5.

However, in order to be able to achieve an adequate pharmacological action of the 2-oxindole-1-carboxamide derivative, a composition for intramuscular injection must contain not less than 20-150 mg of agent per ml of solution for injection. Given the low solubility of only about 2 mg/ml, it would then be necessary to administer not less than 10 ml of solution for injection. However, it is no longer possible to administer a volume as large as this intramuscularly. It is therefore an object of the present invention to provide solutions for injection whose content of 2-oxindole-1-carboxamide derivatives is sufficiently high.

It has been found that a suspension of the agent in an oily vehicle is unsuitable as formulation for intramuscular administration because the absorption of the agent is retarded by the oily vehicle.

It has been found, surprisingly, that the molecular-disperse solubiliser polyvinylpyrrolidone results in a decisive improvement in the solubility of the 2-oxindole-1-carboxamide derivatives in water. However, since the use of polyvinylpyrrolidone for intramuscular administration entails problems in the relatively high concentration range, an administration unit suitable for intramuscular administration ought to contain no more than a total of 50 mg of polyvinylpyrrolidone.

It has now been found within the scope of the present invention that a combination of up to about 50 mg of polyvinylpyrrolidone per administration unit with either polyethylene glycol or N-methylglucamine results in sufficient solubility of the 2-oxindole-1-carboxamide derivative in water to prepare a solution for injection which contains 20-150 mg of 2-oxindole-1-carboxamide derivative per ml of solution for injection.

Hence the present invention relates to pharmaceutical compositions which are intended for injection and which contain a 2-oxindole-1-carboxamide derivative and have as solubiliser a combination of a) polyvinylpyrrolidone and b) polyethylene glycol and/or N-methylglucamine. It is preferable to employ a combination of polyvinylpyrrolidone and either polyethylene glycol or N-methylglucamine as solubiliser for this purpose. A combination of the components polyvinylpyrrolidone, polyethylene glycol and N-methylglucamine can also be used.

The 2-oxindole-1-carboxamide derivatives which can be employed within the scope of the present invention are described in detail in US Patent 4 556 672; express reference is hereby made to this citation.

However, the 2-oxindole-1-carboxamide derivatives which are preferably employed within the scope of the present invention are the compounds of the formula I 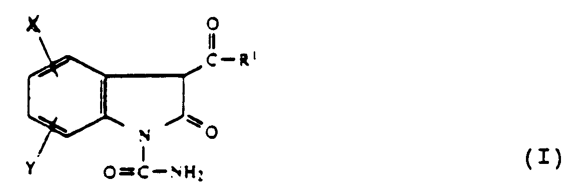
or pharmaceutically acceptable salts thereof, in which Y is hydrogen or chlorine, and X means 5-chloro, 6-chloro, 5-fluoro, 6-fluoro, 5-trifluoromethyl and 6-trifluoromethyl, and in which R¹ is benzyl, 2-furyl, 2-thienyl, (2-furyl)methyl or (2-thienyl)methyl.

2-Oxindole-1-carboxamide derivatives which are particularly preferably employed are those of the following formula (II) 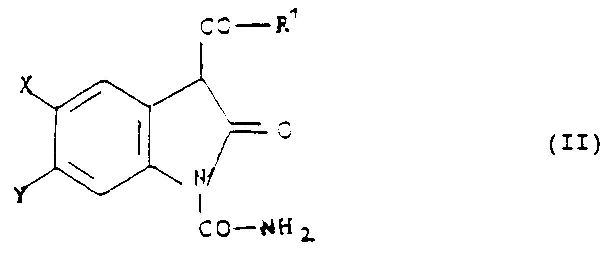
in which X, Y and R¹ have the following meanings:
a) X = Cl, Y = H, R¹ = 2-thienyl, or
b) X = F, Y = Cl, R¹ = 2-thienyl or
c) X = H, Y = Cl and R¹ = benzyl.

Very particularly preferably employed as 2-oxindole-1-carboxamide derivative within the scope of the present invention is the anhydrous polymorphous form A of 5-chloro-3-(2-thenoyl)oxindole-1-carboxamide sodium salt. The corresponding acid can also be employed. As illustration, the corresponding structural formula is indicated below: 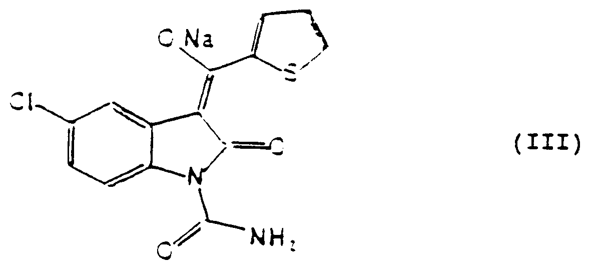
Aqueous solutions which have been prepared using polyethylene glycol and N-methylglucamine are, as a rule, not very stable chemically. Thus, the pharmaceutical compositions according to the invention are within the scope of the present invention subjected, preferably after the preparation of the solutions, to a freeze-drying process in order to obtain a product which can be stored.

Polyvinylpyrrolidone which preferably has an average molecular weight of 2,000-5,000 is used as essential component of the solubiliser system. In a preferred manner, the concentration of polyvinylpyrrolidone is chosen so that a concentration of 50 mg/ml of finished solution for injection is not exceeded.

After freeze-drying of the prepared solution, the pharmaceutical composition preferably contains 5-20% by weight and particularly preferably 7-16% by weight polyvinylpyrrolidone.

Polyethylene glycol which preferably has an average molecular weight between 1,000 and 6,000 can be employed as second component of the solubiliser system. The finished solution for injection ought preferably to contain not more than 350 mg of polyethylene glycol per ml of finished solution for injection. After freeze-drying of the freshly prepared solution, the lyophilised composition preferably contains 50-80% by weight and particularly preferably 60-80% by weight polyethylene glycol.

As alternative to the polyethylene glycol, it is possible to employ as second component of the solubiliser system N-methylglucamine, in which case the concentration of this component is preferably not more than 150 mg of N-methylglucamine per ml of solution for injection. After freeze-drying of the freshly prepared solution, the pharmaceutical composition preferably contains 30-50% by weight and particularly preferably 35-45% by weight N-methylglucamine.

The second component in the solubiliser system which is preferably employed within the scope of the present invention is either polyethylene glycol or N-methylglucamine. However, it is likewise possible to use a combination of these two components.

After freeze-drying of the freshly prepared solutions, the pharmaceutical compositions according to the invention preferably contain 15-55% by weight and particularly preferably 15-40% by weight of 2-oxindole-1-carboxamide derivative.

It has been found, surprisingly, that the lyophilisates according to the invention have a dissolution time of less than 1 minute; this makes straight-forward administration by the treating physician possible without a lengthy wait. It has also been found that the compositions according to the invention are stable when the lyophilisate is stored at temperatures below 30°C. The advantage of this is that storage in a refrigerator is unnecessary, at least when the outside temperatures are not too high.

The pharmaceutical compositions according to the invention can be dissolved without difficulty by adding water, and the solution for injection prepared in this way can be administered parenterally without difficulty.

Furthermore, the pharmaceutical compositions according to the invention can contain customarily used additives known per se. As an alternative to this, additives of this type can also be incorporated in the water used to dissolve the pharmaceutical compositions according to the invention.

### Example 1

129.1 g of N-methylglucamine and 50 g of polyvinylpyrrolidone were dissolved in 2 litres of distilled water. To this solution were added, while stirring, 150 g of 5-chloro-3-(2-thenoyl)oxindole-1-carboxamide, and the pH was adjusted to pH 8.5 with 130 ml of 1 N HCl. After the 2-oxindole-1-carboxamide derivative had dissolved the total volume was made up to 2,500 ml with distilled water. After sterilisation by filtration, 2 ml portions were dispensed into vials and lyophilised in a freeze-drying system.

### Example 2

80 g of polyethylene glycol and 10 g of polyvinylpyrrolidone were dissolved in 200 g of distilled water. To this solution were added 4 mg of NaOH. 26 g of 5-chloro-3-(2-thenoyl)oxindole-1-carboxamide sodium salt were dissolved in this solution while stirring vigorously, then the total volume was made up to 400 ml with water. After sterilisation by filtration, 2 ml portions were dispensed into vials and lyophilised in a freeze-drying system.

## Claims (Claims for the following Contracting State(s): AT, BE, CH, DE, DK, FR, GB, IT, LI, NL, PT, SE)

1. Pharmaceutical composition which is intended for injection and which contains a 2-oxindole-1-carboxamide derivative, characterised in that it contains as solubiliser a combination of
a) polyvinylpyrrolidone and
b) polyethylene glycol and/or N-methylglucamine.

2. Pharmaceutical composition according to Claim 1, characterised in that the 2-oxindole-1-carboxamide derivative is a compound of the formula I 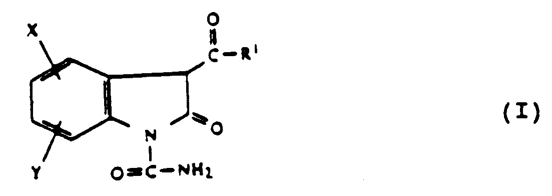 or a pharmaceutically acceptable salt thereof, in which Y is hydrogen or chlorine, and X means 5-chloro, 6-chloro, 5-fluoro, 6-fluoro, 5-trifluoromethyl and 6-trifluoromethyl, and in which R¹ is benzyl, 2-furyl, 2-thienyl, (2-furyl)methyl or (2-thienyl)methyl.

3. Pharmaceutical composition according to Claim 2, characterised in that the 2-oxindole-1-carboxamide derivative has the formula II 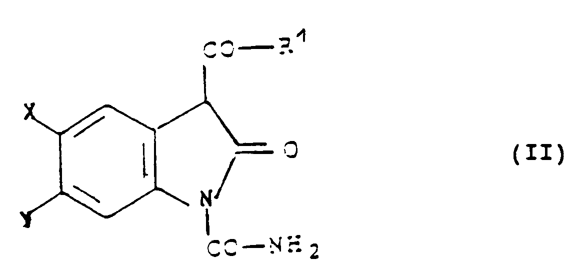 in which the substituents X, Y and R¹ have the following meanings:
a) X = Cl, Y = H, R¹ = 2-thienyl, or
b) X = F, Y = Cl, R¹ = 2-thienyl or
c) X = H, Y = Cl and R¹ = benzyl.

4. Pharmaceutical composition according to any of the preceding claims, characterised in that 5-chloro-3-(2-thenoyl)oxindole-1-carboxamide in the form of the acid or as anhydrous polymorphous form A of the sodium salt is employed as 2-oxindole-1-carboxamide derivative.

5. Pharmaceutical composition according to any of the preceding claims, characterised in that it is in the form of a lyophilisate.

6. Pharmaceutical composition according to Claim 5, characterised in that it contains 5-20% by weight polyvinylpyrrolidone.

7. Pharmaceutical composition according to Claim 5, characterised in that it contains 7-16% by weight polyvinylpyrrolidone.

8. Pharmaceutical composition according to Claim 6 or 7, characterised in that the polyvinylpyrrolidone has an average molecular weight of 2,000-5,000.

9. Pharmaceutical composition according to any of Claims 5 to 8, characterised in that it contains 50-80% by weight polyethylene glycol.

10. Pharmaceutical composition according to Claim 9, characterised in that it contains 60-80% by weight polyethylene glycol.

11. Pharmaceutical composition according to either of Claims 9 or 10, characterised in that the polyethylene glycol has an average molecular weight of 1,000-6,000.

12. Pharmaceutical composition according to any of Claims 5 to 8, characterised in that it contains 30-50% by weight N-methylglucamine.

13. Pharmaceutical composition according to Claim 12, characterised in that it contains 35-45% by weight N-methylglucamine.

14. Pharmaceutical composition according to either of Claims 12 or 13, characterised in that it contains no polyethylene glycol.

15. Pharmaceutical composition according to any of Claims 1 to 14, characterised in that it contains 15-55% by weight of the 2-oxindole-1-carboxamide derivative.

16. Pharmaceutical composition according to Claim 15, characterised in that it contains 15-40% by weight of the 2-oxindole-1-carboxamide derivative.

17. Pharmaceutical composition according to any of Claims 1 to 4, characterised in that it is in the form of a solution for injection which can be administered parenterally.

## Claims (Claims for the following Contracting State(s): ES, GR)

1. Process for preparing a pharmaceutical composition which is intended for injection and which contains a 2-oxindole-1-carboxamide derivative, characterized in that the 2-oxindole-1-carboxamide derivative is solubilized by a combination of
a) polyvinylpyrrolidone and
b) polyethylene glycol and/or N-methylglucamine.

2. Process according to Claim 1, characterised in that the 2-oxindole-1-carboxamide derivative is a compound of the formula I 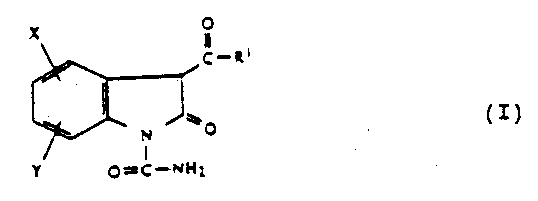 or a pharmaceutically acceptable salt thereof, in which Y is hydrogen or chlorine, and X means 5-chloro, 6-chloro, 5-fluoro, 6-fluoro, 5-trifluoromethyl and 6-trifluoromethyl, and in which R¹ is benzyl, 2-furyl, 2-thienyl, (2-furyl)methyl or (2-thienyl)methyl.

3. Process according to Claim 2, characterised in that the 2-oxindole-1-carboxamide derivative has the formula II 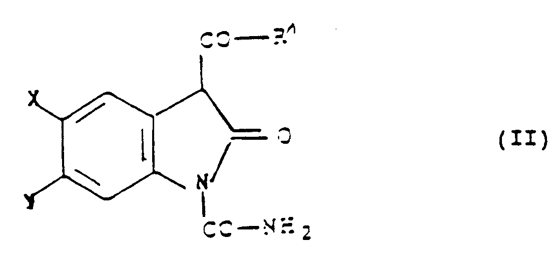 in which the substituents X, Y and R¹ have the following meanings:
a) X = Cl, Y = H, R¹ = 2-thienyl, or
b) X = F, Y = Cl, R¹ = 2-thienyl or
c) X = H, Y = Cl and R¹ = benzyl.

4. Process according to any of the preceding claims, characterised in that 5-chloro-3-(2-thenoyl)oxindole-1-carboxamide in the form of the acid or as anhydrous polymorphous form A of the sodium salt is employed as 2-oxindole-1-carboxamide derivative.

5. Process according to any of the preceding claims, characterised in that it further comprises freeze-drying the pharmaceutical composition.

6. Process according to Claim 5, characterised in that the pharmaceutical composition contains 5-20% by weight polyvinylpyrrolidone.

7. Process according to Claim 5, characterised in that the pharmaceutical composition contains 7-16% by weight polyvinylpyrrolidone.

8. Process according to Claim 6 or 7, characterised in that the polyvinylpyrrolidone has an average molecular weight of 2,000-5,000.

9. Process according to any of Claims 5 to 8, characterised in that the pharmaceutical composition contains 50-80% by weight polyethylene glycol.

10. Process according to Claim 9, characterised in that the pharmaceutical composition contains 60-80% by weight polyethylene glycol.

11. Process according to either of Claims 9 or 10, characterised in that the polyethylene glycol has an average molecular weight of 1,000-6,000.

12. Process according to any of Claims 5 to 8, characterised in that the pharmaceutical composition contains 30-50% by weight N-methylglucamine.

13. Process according to Claim 12, characterised in that the pharmaceutical composition contains 35-45% by weight N-methylglucamine.

14. Process according to either of Claims 12 or 13, characterised in that the pharmaceutical composition contains no polyethylene glycol.

15. Process according to any of Claims 1 to 14, characterised in that the pharmaceutical composition contains 15-55% by weight of the 2-oxindole-1-carboxamide derivative.

16. Process according to Claim 15, characterized in that the pharmaceutical composition contains 15-40% by weight of the 2-oxindole-1-carboxamide derivative.

17. Process according to any of Claims 1 to 4, characterized in that the pharmaceutical composition is in the form of a solution for injection which can be administered parenterally.

## Patentansprüche (Patentansprüche für folgende(n) Vertragsstaat(en): AT, BE, CH, DE, DK, FR, GB, IT, LI, NL, PT, SE)

1. Pharmazeutische, zur Injektion bestimmte Zusammensetzung, die ein 2-Oxindol-1-carboxamid-Derivat enthält, dadurch gekennzeichnet, daß sie als Lösungsvermittler eine Kombination von
a) Polyvinylpyrrolidon und
b) Polyethylenglycol und/oder N-Methylglucamin
enthält.

2. Pharmazeutische Zusammensetzung gemäß Anspruch 1, dadurch gekennzeichnet, daß das 2-Oxindol-1-carboxamid-Derivat eine Verbindung der Formel I 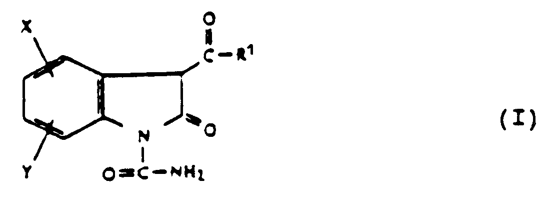 ist oder ein pharmazeutisch akzeptables Salz davon, worin Y Wasserstoff oder Chlor ist, X 5-Chlor, 6-Chlor, 5-Fluor, 6-Fluor, 5-Trifluormethyl und 6-Trifluormethyl bedeutet und worin R¹ Benzyl, 2-Furyl, 2-Thienyl, (2-Furyl)methyl oder (2-Thienyl)methyl ist.

3. Pharmazeutische Zusammensetzung gemäß Anspruch 2, dadurch gekennzeichnet, daß das 2-Oxindol-1-carboxamid-Derivat die Formel II 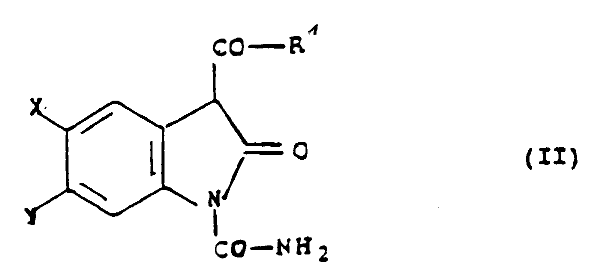 hat, worin die Substituenten X, Y und R¹ die folgenden Bedeutungen haben:
a) X = Cl, Y = H, R¹ = 2-Thienyl, oder
b) X = F, Y = Cl, R¹ = 2-Thienyl oder
c) X = H, Y = Cl und R¹ = Benzyl.

4. Pharmazeutische Zusammensetzung gemäß einem der vorhergehenden Ansprüche, dadurch gekennzeichnet, daß 5-Chlor-3-(2-thenoyl)oxindol-1-carboxamid in Form der Säure oder als wasserfreie, polymorphe Form A des Natriumsalzes eingesetzt wird als 2-Oxindol-1-carboxamid-Derivat.

5. Pharmazeutische Zusammensetzung gemäß einem der vorhergehenden Ansprüche, dadurch gekennzeichnet, daß sie in Form eines Lyophilisats vorliegt.

6. Pharmazeutische Zusammensetzung gemäß Anspruch 5, dadurch gekennzeichnet, daß sie 5-20 Gew.-% Polyvinylpyrrolidon enthält.

7. Pharmazeutische Zusammensetzung gemäß Anspruch 5, dadurch gekennzeichnet, daß sie 7-16 Gew.-% Polyvinylpyrrolidon enthält.

8. Pharmazeutische Zusammensetzung gemäß Anspruch 6 oder 7, dadurch gekennzeichnet, daß das Polyvinylpyrrolidon ein durchschnittliches Molekulargewicht von 2.000-5.000 hat.

9. Pharmazeutische Zusammensetzung gemäß einem der Ansprüche 5 bis 8, dadurch gekennzeichnet, daß sie 50-80 Gew.-% Polyethylenglycol enthält.

10. Pharmazeutische Zusammensetzung gemäß Anspruch 9, dadurch gekennzeichnet, daß sie 60-80 Gew.-% Polyethylenglycol enthält.

11. Pharmazeutische Zusammensetzung gemäß einem der Ansprüche 9 oder 10, dadurch gekennzeichnet, daß das Polyethylenglycol ein durchschnittliches Molekulargewicht von 1.000-6.000 hat.

12. Pharmazeutische Zusammensetzung gemäß einem der Ansprüche 5 bis 8, dadurch gekennzeichnet, daß sie 30-50 Gew.-% N-Methylglucamin enthält.

13. Pharmazeutische Zusammensetzung gemäß Anspruch 12, dadurch gekennzeichnet, daß sie 35-45 Gew.-% N-Methylglucamin enthält.

14. Pharmazeutische Zusammensetzung gemäß einem der Ansprüche 12 oder 13, dadurch gekennzeichnet, daß sie kein Polyethylenglycol enthält.

15. Pharmazeutische Zusammensetzung gemäß einem der Ansprüche 1 bis 14, dadurch gekennzeichnet, daß sie 15-55 Gew.-% des 2-Oxindol-1-carboxamid-Derivates enthält.

16. Pharmazeutische Zusammensetzung gemäß Anspruch 15, dadurch gekennzeichnet, daß sie 15-40 Gew.-% des 2-Oxindol-1-carboxamid-Derivates enthält.

17. Pharmazeutische Zusammensetzung gemäß einem der Ansprüche 1 bis 4, dadurch gekennzeichnet, daß sie in Form einer Injektionslösung vorliegt, die parenteral verabreicht werden kann.

## Patentansprüche (Patentansprüche für folgende(n) Vertragsstaat(en): ES, GR)

1. Verfahren zur Herstellung einer pharmazeutischen, zur Injektion bestimmten Zusammensetzung, die ein 2-Oxindol-1-carboxamid-Derivat enthält, dadurch gekennzeichnet, daß das 2-Oxindol-carboxamid-Derivat löslich gemacht wird durch eine Kombination von
a) Polyvinylpyrrolidon und
b) Polyethylenglycol und/oder N-Methylglucamin.

2. Verfahren gemäß Anspruch 1, dadurch gekennzeichnet, daß das 2-Oxindol-1-carboxamid-Derivat eine Verbindung der Formel I 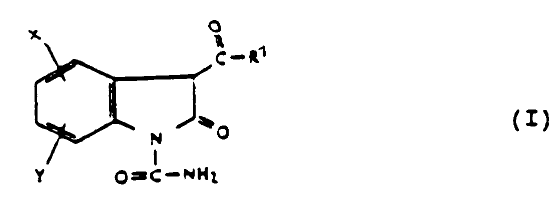 ist oder ein pharmazeutisch akzeptables Salz davon, worin Y Wasserstoff oder Chlor ist, X 5-Chlor, 6-Chlor, 5-Fluor, 6-Fluor, 5-Trifluormethyl und 6-Trifluormethyl bedeutet und worin R¹ Benzyl, 2-Furyl, 2-Thienyl, (2-Furyl)methyl oder (2-Thienyl)methyl ist.

3. Verfahren gemäß Anspruch 2, dadurch gekennzeichnet, daß das 2-Oxindol-1-carboxamid-Derivat die Formel II 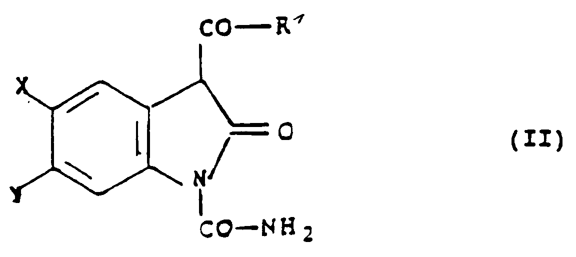 hat, worin die Substituenten X, Y und R¹ die folgenden Bedeutungen haben:
a) X = Cl, Y = H, R¹ = 2-Thienyl, oder
b) X = F, Y = Cl, R¹ = 2-Thienyl oder
c) X = H, Y = Cl und R¹ = Benzyl.

4. Verfahren gemäß einem der vorhergehenden Ansprüche, dadurch gekennzeichnet, daß 5-Chlor-3-(2-thenoyl)oxindol-1-carboxamid in Form der Säure oder als wasserfreie, polymorphe Form A des Natriumsalzes eingesetzt wird als 2-Oxindol-1-carboxamid-Derivat.

5. Verfahren gemäß einem der vorhergehenden Ansprüche, dadurch gekennzeichnet, daß es weiterhin die Gefriertrocknung der pharmazeutischen Zusammensetzung umfaßt.

6. Verfahren gemäß Anspruch 5, dadurch gekennzeichnet, daß die pharmazeutische Zusammensetzung 5-20 Gew.-% Polyvinylpyrrolidon enthält.

7. Verfahren gemäß Anspruch 5, dadurch gekennzeichnet, daß die pharmazeutische Zusammensetzung 7-16 Gew.-% Polyvinylpyrrolidon enthält.

8. Verfahren gemäß Anspruch 6 oder 7, dadurch gekennzeichnet, daß das Polyvinylpyrrolidon ein durchschnittliches Molekulargewicht von 2.000-5.000 hat.

9. Verfahren gemäß einem der Ansprüche 5 bis 8, dadurch gekennzeichnet, daß die pharmazeutische Zusammensetzung 50-80 Gew.-% Polyethylenglycol enthält.

10. Verfahren gemäß Anspruch 9, dadurch gekennzeichnet, daß die pharmazeutische Zusammensetzung 60-80 Gew.-% Polyethylenglycol enthält.

11. Verfahren gemäß einem der Ansprüche 9 oder 10, dadurch gekennzeichnet, daß das Polyethylenglycol ein durchschnittliches Molekulargewicht von 1.000-6.000 hat.

12. Verfahren gemäß einem der Ansprüche 5 bis 8, dadurch gekennzeichnet, daß die pharmazeutische Zusammensetzung 30-50 Gew.-% N-Methylglucamin enthält.

13. Verfahren gemäß Anspruch 12, dadurch gekennzeichnet, daß die pharmazeutische Zusammensetzung 35-45 Gew.-% N-Methylglucamin enthält.

14. Verfahren gemäß einem der Ansprüche 12 oder 13, dadurch gekennzeichnet, daß die pharmazeutische Zusammensetzung kein Polyethylenglycol enthält.

15. Verfahren gemäß einem der Ansprüche 1 bis 14, dadurch gekennzeichnet, daß die pharmazeutische Zusammensetzung 15-55 Gew.-% des 2-Oxindol-1-carboxamid-Derivates enthält.

16. Verfahren gemäß Anspruch 15, dadurch gekennzeichnet, daß die pharmazeutische Zusammensetzung 15-40 Gew.-% des 2-Oxindol-1-carboxamid-Derivates enthält.

17. Verfahren gemäß einem der Ansprüche 1 bis 4, dadurch gekennzeichnet, daß die pharmazeutische Zusammensetzung in Form einer Injektionslösung vorliegt, die parenteral verabreicht werden kann.

## Revendications (Revendications pour l'(les) Etat(s) contractant(s) suivant(s): AT, BE, CH, DE, DK, FR, GB, IT, LI, NL, PT, SE)

1. Composition pharmaceutique destinée à être injectée et qui contient un dérivé du 2-oxindole-1-carboxamide, caractérisée en ce qu'elle contient en tant qu'agent de solubilisation, une combinaison
a) de polyvinylpyrrolidone et
b) de polyéthylèneglycol et/ou de N-méthylglucamine.

2. Composition pharmaceutique suivant la revendication 1, caractérisée en ce que le dérive du 2-oxindole-1-carboxamide est un composé de la formule I 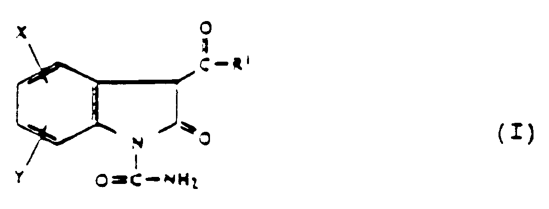 ou un sel pharmaceutiquement acceptable de celui-ci, dans laquelle
Y représente l'hydrogène ou le chlore, et X désigne 5-chloro, 6-chloro, 5-fluoro, 6-fluoro, 5-trifluorométhyle et 6-trifluorométhyle, et dans laquelle R¹ représente le radical benzyle, 2-furyle, 2-thiényle, (2-furyl)méthyle ou (2-thiényl)méthyle.

3. Composition pharmaceutique suivant la revendication 2, caractérisée en ce que le dérivé du 2-oxindole-1-carboxamide possède la formule II 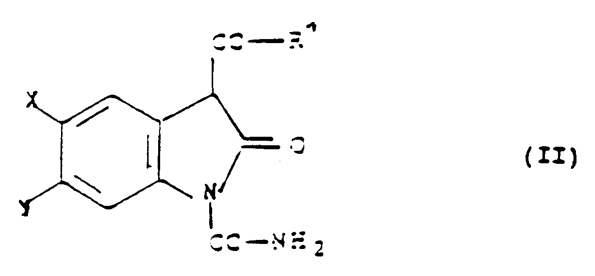 dans laquelle les substituants X, Y et R¹ ont les significations qui suivent :
a) X = Cl, Y = H, R¹ = 2-thiényle ou
b) X = F, Y = Cl, R¹ = 2-thiényle ou
c) X = H, Y = Cl et R¹ = benzyle.

4. Composition pharmaceutique suivant l'une quelconque des revendications précédentes, caractérisée en ce que l'on utilise le 5-chloro-3-(2-thénoyl)oxindole-1-carboxamide sous sa forme acide ou sous sa forme A anhydre polymorphe du sel de sodium à titre de dérivé du 2-oxindole-1-carboxamide.

5. Composition pharmaceutique suivant l'une quelconque des revendications précédentes, caractérisée en ce qu'elle se présente sous forme lyophilisée.

6. Composition pharmaceutique suivant la revendication 5, caractérisée en ce qu'elle contient de 5 à 20% en poids de polyvinylpyrrolidone.

7. Composition pharmaceutique suivant la revendication 5, caractérisée en ce qu'elle contient de 7 à 16% en poids de polyvinylpyrrolidone.

8. Composition pharmaceutique suivant la revendication 6 ou 7, caractérisée en ce que la polyvinylpyrrolidone possède un poids moléculaire moyen qui varie de 2000 à 5000.

9. Composition pharmaceutique suivant l'une quelconque des revendications 5 à 8, caractérisée en ce qu'elle contient de 50 à 80% en poids de polyéthylèneglycol.

10. Composition pharmaceutique suivant la revendication 9, caractérisée en ce qu'elle contient de 60 à 80% en poids de polyéthylèneglycol.

11. Composition pharmaceutique suivant la revendication 9 ou 10, caractérisée en ce que polyéthylèneglycol possède un poids moléculaire moyen qui varie de 1000 à 6000.

12. Composition pharmaceutique suivant l'une quelconque des revendications 5 à 8, caractérisée en ce qu'elle contient de 30 à 50% en poids de N-méthylglucamine.

13. Composition pharmaceutique suivant la revendication 12, caractérisée en ce qu'elle contient de 35 à 45% en poids de N-méthylglucamine.

14. Composition pharmaceutique suivant la revendication 12 ou 13, caractérisée en ce qu'elle est exempte de polyéthylèneglycol.

15. Composition pharmaceutique suivant l'une quelconque des revendications 1 à 14, caractérisée en ce qu'elle contient de 15 à 55% en poids du dérivé du 2-oxindole-1-carboxamide.

16. Composition pharmaceutique suivant la revendication 15, caractérisée en ce qu'elle contient de 15 à 40% en poids du dérivé du 2-oxindole-1-carboxamide.

17. Composition pharmaceutique suivant l'une quelconque des revendications 1 à 4, caractérisée en ce qu'elle se présente sous la forme d'une solution injectable qui peut être administrée par voie parentérale.

## Revendications (Revendications pour l'(les) Etat(s) contractant(s) suivant(s): ES, GR)

1. Procédé de préparation d'une composition pharmaceutique destinée à être injectée et qui contient un dérivé du 2-oxindole-1-carboxamide, caractérisé en ce qu'il contient en tant qu'agent de solubilisation, une combinaison
a) de polyvinylpyrrolidone et
b) de polyéthylèneglycol et/ou de N-méthylglucamine.

2. Procédé suivant la revendication 1, caractérisé en ce que le dérivé du 2-oxindole-1-carboxamide est un composé de la formule I 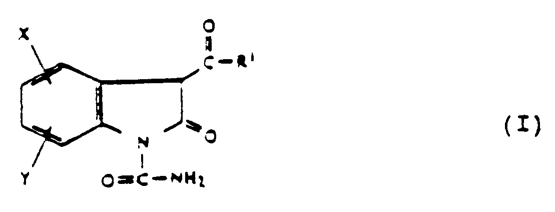 ou un sel pharmaceutiquement acceptable de celui-ci, dans laquelle
Y représente l'hydrogène ou le chlore, et X désigne 5-chloro, 6-chloro, 5-fluoro, 6-fluoro, 5-trifluorométhyle et 6-trifluorométhyle, et dans laquelle R¹ représente le radical benzyle, 2-furyle, 2-thiényle, (2-furyl)méthyle ou (2-thiényl)méthyle.

3. Procédé suivant la revendication 2, caractérisé en ce que le dérivé du 2-oxindole-1-carboxamide possède la formule II 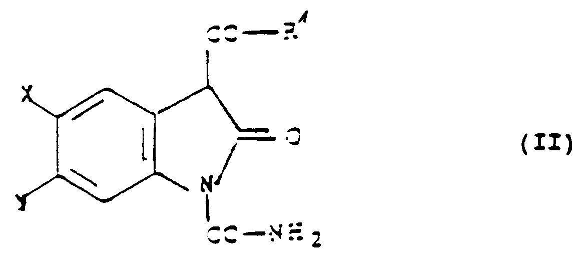 dans laquelle les substituants X, Y et R¹ ont les significations qui suivent :
a) X = Cl, Y = H, R¹ = 2-thiényle ou
b) X = F, Y = Cl, R¹ = 2-thiényle ou
c) X = H, Y = Cl et R¹ = benzyle.

4. Procédé suivant l'une quelconque des revendications précédentes, caractérisé en ce que l'on utilise le 5-chloro-3-(2-thénoyl)oxindole-1-carboxamide sous sa forme acide ou sous sa forme A anhydre polymorphe du sel de sodium à titre de dérivé du 2-oxindole-1-carboxamide.

5. Procédé suivant l'une quelconque des revendications précédentes, caractérisé en ce qu'il comprend en outre, une étape de lyophilisation de la composition pharmaceutique.

6. Procédé suivant la revendication 5, caractérisé en ce qu'il contient de 5 à 20% en poids de polyvinylpyrrolidone.

7. Procédé suivant la revendication 5, caractérisé en ce qu'il contient de 7 à 16% en poids de polyvinylpyrrolidone.

8. Procédé suivant la revendication 6 ou 7, caractérisé en ce que la polyvinylpyrrolidone possède un poids moléculaire moyen qui varie de 2000 à 5000.

9. Procédé suivant l'une quelconque des revendications 5 à 8, caractérisé en ce qu'il contient de 50 à 80% en poids de polyéthylèneglycol.

10. Procédé suivant la revendication 9, caractérisé en ce qu'il contient de 60 à 80% en poids de polyéthylèneglycol.

11. Procédé suivant la revendication 9 ou 10, caractérisé en ce que polyéthylèneglycol possède un poids moléculaire moyen qui varie de 1000 à 6000.

12. Procédé suivant l'une quelconque des revendications 5 à 8, caractérisé en ce qu'il contient de 30 à 50% en poids de N-méthylglucamine.

13. Procédé suivant la revendication 12, caractérisé en ce qu'il contient de 35 à 45% en poids de N-méthylglucamine.

14. Procédé suivant la revendication 12 ou 13, caractérisé en ce qu'il est exempt de polyéthylèneglycol.

15. Procédé suivant l'une quelconque des revendications 1 à 14, caractérisé en ce qu'il contient de 15 à 55% en poids du dérivé du 2-oxindole-1-carboxamide.

16. Procédé suivant la revendication 15, caractérisé en ce qu'il contient de 15 à 40% en poids du dérivé du 2-oxindole-1-carboxamide.

17. Procédé suivant l'une quelconque des revendications 1 à 4, caractérisé en ce qu'il se présente sous la forme d'une solution injectable qui peut être administrée par voie parentérale.
